# EUROPEAN PATENT SPECIFICATION

(11) **EP 1 761 206 B1**
(45) Date of publication and mention of the grant of the patent: **18.03.2020**
(21) Application number: 05778125.4
(22) Date of filing: 23.06.2005
(51) Int. Cl.: A61F 2/966, A61F 2/95, A61F 2/97, A61F 2/958

(54) **DEVICES AND METHODS FOR CONTROLLING EXPANDABLE PROSTHESES DURING DEPLOYMENT**
VORRICHTUNGEN UND VERFAHREN ZUR STEUERUNG EXPANDIERBARER PROTHESEN WÄHREND DER POSITIONIERUNG
DISPOSITIFS ET PROCEDES POUR LE CONTROLE DE PROTHESES EXPANSIBLES LORS DE LEUR DEPLOIEMENT

(30) Priority: 28.06.2004 US 879949; 30.09.2004 US 957079
(43) Date of publication of application: 14.03.2007
(73) Proprietor: J.W. Medical Systems Ltd., Weihai, Shandong 264200 (CN)
(72) Inventor: PLAIN, Henry, Atherton, CA 94027 (US); ANDREAS, Bernard, Redwood City, CA 94062 (US); SNOW, David, W., San Carlos, CA 94070 (US)
(74) Representative: Avidity IP
(86) International application number: PCT/US2005/024931
(87) International publication number: WO 2006/005082

(56) References cited:
- EP-A1- 1 097 728
- US-A- 5 824 041
- US-A- 5 833 694
- US-A- 5 833 694
- US-A1- 2004 093 061

## Description

### BACKGROUND OF THE INVENTION

Stents are tubular prostheses designed for implantation in a vessel to maintain patency of the vessel lumen. Stents are used in various vessels throughout the body, including the coronary arteries, femoral arteries, iliac arteries, renal artery, carotid artery, vascular grafts, biliary ducts, trachea, and urethra, to name some examples. Stents are typically implanted by means of long and flexible delivery catheters that carry the stents in a compact, collapsed shape to the treatment site and then deploy the stents into the vessel. In some applications, balloon expandable stents are used. These stents are made of a malleable metal such as stainless steel or cobalt chromium and are expanded by means of a balloon on the tip of the delivery catheter to plastically deform the stent into contact with the vessel wall. In other applications, self-expanding stents are used. These are made of a resilient material that can be collapsed into a compact shape for delivery via catheter and that will self-expand into contact with the vessel when deployed from the catheter. Materials commonly used for self-expanding stents include stainless steel and elastic or superelastic alloys such as nickel titanium (Nitinol™).

While self-expanding stents have demonstrated promise in various applications, such stents face a number of challenges. One such challenge is that in some cases the disease in a vessel may be so extensive that a stent of very long length, e.g. 30-200 mm, is called for. Currently available stents are typically less than 30 mm in length, and suffer from excessive stiffness if made longer. Such stiffness is particularly problematic in peripheral vessels such as the femoral arteries, where limb movement requires a high degree of flexibility in any stent implanted in such vessels.

To overcome the stiffness problem, the idea of deploying multiple shorter stents end-to-end has been proposed. However, this approach has suffered from several drawbacks. First, currently available delivery catheters are capable of delivering only a single stent per catheter. In order to place multiple stents, multiple catheters must be inserted, removed and exchanged, heightening risks, lengthening procedure time, raising costs, and causing excessive material waste. In addition, the deployment of multiple stents end-to-end suffers from the inability to accurately control stent placement and the spacing between stents. This results in overlap of adjacent stents and/or excessive space between stents, which is thought to lead to complications such as restenosis, the renarrowing of a vessel following stent placement. With self-expanding stents the problem is particularly acute because as the stent is released from the catheter, its resiliency tends to cause it to eject or "watermelon seed" distally from the catheter tip by an unpredictable distance. During such deployment, the stent may displace not only axially but rotationally relative to the delivery catheter resulting in inaccurate, uncontrollable, and unpredictable stent placement.
For background art, concerning known features of the main claim, the reader is directed to the disclosure and drawings in the publication EP 1097 728 A in which a stent or stent graft locating device comprises a number of leading wires a pushing rod and a sheath for loading the pushing rod.

Interleaving stents or stent segments such as those disclosed in co-pending application Serial No. 10/738,666, filed December 16, 2003, (US2004/0186551A1), present even greater challenges to conventional delivery systems. Interleaving stents have axially extending elements on each end of the stent that interleave with similar structures on an adjacent stent. Such interleaving minimizes the gap between adjacent stents and increases vessel wall coverage to ensure adequate scaffolding and minimize protrusion of plaque from the vessel wall. However, such interleaving requires that the relative rotational as well as axial positions of the adjacent stents be maintained during deployment to avoid metal overlap and excessive gaps between stents. Conventional delivery systems suffer from the inability to control both the axial and rotational positions of self-expanding stents as they are deployed.

What are needed, therefore, are stents and stent delivery systems that overcome the foregoing problems. In particular, the stents and stent delivery systems should facilitate stenting of long vascular regions of various lengths without requiring the use of multiple catheters. Such stents and delivery systems should also provide sufficient flexibility for use in peripheral vessels and other regions where long and highly flexible stents might be required. In addition, the stents and stent delivery systems should enable the delivery of multiple stents of various lengths to one or more treatment sites using a single catheter without requiring catheter exchanges. Further, the stents and stent delivery systems should facilitate accurate and repeatable control of stent placement and inter-stent spacing to enable deployment of multiple self-expanding stents end-to-end in a vessel at generally constant spacing and without overlap. Moreover, the stents and delivery systems should enable the deployment of interleaving stents or stent segments with precision and control over both the axial spacing and rotational position of each stent or segment.

### BRIEF SUMMARY OF THE INVENTION

The present invention provides a stent delivery system, that enables the precise and controllable delivery of multiple stents using a single delivery catheter. The stent delivery system provides for the precise control of stent placement so that inter-stent spacing can be maintained at a constant and optimum distance. In some stent delivery catheter embodiments, both axial and rotational displacement of the stents relative to the delivery catheter can be controlled during deployment, enabling the delivery of multiple stents that interleave with one another without overlap. The stent delivery systemof the invention further enables the length of prostheses to be customized *in situ* to match the length of the site to be treated. The invention is particularly useful for delivery of self-expanding stents, but balloon expandable stents are also contemplated within the scope of the invention. The present catheters are well- suited to delivery of stents to the coronary arteries and to peripheral vessels such as the popliteal, femoral, tibial, iliac, renal, and carotid arteries. The present catheters are further useful for delivery of stents to other vessels including biliary, neurologic, urinary, reproductive, intestinal, pulmonary, and others, as well as for delivery of other types of stents to various anatomical regions, wherever precise control of stent deployment is desirable.
According to this invention there is provided a stent delivery catheter as defined in claim 1. Preferred and optional features are to be found amongst the sub-claims.

In a first aspect of the invention, a stent delivery catheter includes an outer shaft having a first lumen; a plurality of self-expanding tubular stents carried within the first lumen, the stents being adapted to radially expand upon deployment from the first lumen; a deployment mechanism provided for deploying a selected number of the stents from the first lumen; and a control member interactive with the stents to control their expansion when deployed from the first lumen.

The control member comprises(i) a plurality of axially-extending control wires, the stents being coupled to the wires and axially slidable thereon, the wires being radially deflectable to allow controlled expansion of the stents. The wires may have free distal ends configured to move radially outward as the stents expand. The distal ends of the wires may be retractable into the outer shaft following deployment of the selected number of stents. The stents may have sidewalls with a plurality of openings, the wires being threaded through the openings. The wires may form a loop extending around the outside of the stents and through the inside of the prostheses, wherein the wires can be withdrawn from around the stents following deployment thereof. In such case, at least one end of each wire is releasable to allow the wire to be withdrawn following stent deployment.

The delivery catheter may further comprise an inner shaft disposed in the first lumen, the stents being slidably disposed around the inner shaft, wherein a distal end of each wire is releasably coupled to the inner shaft. A nosecone may be attached to the inner shaft distally of the stents, the distal end of each wire being releasably coupled to the nosecone. The inner shaft may also have an inner lumen and at least one port in communication with the inner lumen, wherein the control wires are slidably disposed through the inner lumen and the port.

The control member may also comprise a sleeve disposed around the stents, the sleeve being expandable to allow their controlled expansion. The sleeve may be elastomeric, an expandable mesh or woven material, or other expandable structure. When expanded, the sleeve may form a cone shape that flares in the distal direction. The sleeve may be slidable relative to the outer shaft. The sleeve may have at least one longitudinal slit therein whereby it expands by splitting at the longitudinal slit. The sleeve may have a pair of opposing edges bordering the longitudinal slit, a cone shape being formed by moving the edges at an angle relative to each other. The sleeve may also have a plurality of longitudinal sections or beams separated by longitudinal slits, the longitudinal sections being deflectable outwardly to allow controlled expansion of the stents. A retainer may be releasably coupled to the longitudinal sections to selectively prevent radial deflection thereof. The retainer may comprise a capsule coupled to an inner shaft slidably disposed through the first lumen, longitudinal sections being received in the capsule.

The deployment mechanism of the delivery catheter may comprise a pushing element slidably disposed in the first lumen, the pushing element being in engagement with at least one of the stents to advance them distally relative to the outer shaft. In preferred embodiments, the stents are self-expandable, made of resilient or shape memory materials such as stainless steel, Nitinol or suitable polymers. Such self- expanding stents are held in an unexpanded state within the outer shaft until deployed therefrom, whereupon they can resiliently expand to an expanded shape in contact with the vessel wall or lesion. The delivery system of the invention will also be useful with balloon expandable stents. In either case, expandable balloons, valve members, and other mechanisms may also be included in the delivery catheter to facilitate stent deployment.

In a further aspect of the invention, the stents are releasably interconnected to each other. In this case, the control member may comprise an interconnection structure on the pushing element, the interconnection structure being releasably coupled to at least one of the stents to resist their distal movement relative to the outer shaft.

In addition to controlling axial position of the stents relative to the delivery catheter and/or to each other during deployment, the control member of the delivery catheter is preferably configured to maintain rotational position of the stents relative to each other. This facilitates the delivery of stents having axially interleaving elements and prevents excessive spacing or overlap between such elements

In an illustrative arrangement, a stent delivery catheter for delivering stents into a vessel lumen may be provided and which comprises an outer shaft having a first lumen; a plurality of self-expanding tubular stents carried within the first lumen, the stents being adapted to radially expand upon deployment from the first lumen; a deployment mechanism for deploying a selected number of the stents from the first lumen; and an anchor member adapted to engage the vessel to limit movement of the outer shaft relative thereto when a stent is being deployed. In one such arrangemnt, the anchor member comprises an expandable member mounted on an inner shaft, the inner shaft being slidably disposed in the first lumen. The expandable member preferably comprises a balloon. The expandable member may be configured to expand within a deployed stent in the vessel lumen. The expandable member is preferably configured to remain expanded within the deployed stent while a second stent is deployed adjacent to the deployed stent. This maintains the relative positions of the deployed stent and the delivery catheter so the second stent is deployed at a predictable distance from the deployed stent.

In another arrangement, a stent delivery catheter for delivering stents into a vessel lumen comprises an outer shaft having a first lumen; a plurality of self-expanding tubular stents carried within the first lumen, the stents being adapted to radially expand upon deployment from the first lumen, each stent comprising a distal portion and proximal portion, the distal portion being configured to expand into engagement with the vessel while the proximal portion is at least partially disposed in the first lumen; and a deployment mechanism for deploying a selected number of the stents from the first lumen. Preferably, the distal portion is configured to engage the vessel prior to deployment of the proximal portion so that the stent remains in a generally constant position relative to the catheter as the proximal portion is deployed.

In one such arrangement, the distal and proximal portions of the stents are interconnected by at least one spring member, the spring member having a retracted shape and an elongated shape and being biased into the retracted shape, wherein deployment of the distal portion into the vessel elongates the spring into the elongated shape. In such a case, the deployment of the proximal portion into the vessel allows the spring to return at least partially to the retracted shape to draw the proximal portion toward the distal portion.

The stent delivery catheter can be used in a method of delivering one or more stents to a treatment site in a vessel, such method not being part of the claimed invention but comprising positioning a delivery catheter at the treatment site, the delivery catheter carrying a plurality of self-expanding stents; selecting a desired number of the stents to deploy; deploying the desired number of stents from the delivery catheter into the vessel, each stent expanding into contact with the vessel upon deployment; and controlling the axial displacement of each of the selected number of stents relative to the delivery catheter during the deployment thereof.

In one arrangement for use in such a method, the axial displacement can be controlled by an expandable sleeve disposed around the desired number of stents . Such method may further include retracting the sleeve from around the stents after the stents have been deployed. The axial displacement may also be controlled by a plurality of wires coupled with the desired number of stents. The wires may be threaded through openings in each of the stents, and may be retracted from them after deployment.

The method of use ,not being part of the claimed invention may further include controlling the rotational displacement of the selected number of stents relative to the delivery catheter and/or relative to each other during the deployment thereof.

The axial displacement of the stents may be controlled by expanding an expandable member in the vessel during deployment of at least a portion of the desired number of stents. Alternatively, the axial displacement may be controlled by first expanding a distal portion of a first of the stents into engagement with the vessel while a proximal portion of the first of the stents remains in the delivery catheter, then expanding the proximal portion of the first of the stents into engagement with the vessel.

As a further alternative, the stents may be releasably interconnected while in the delivery catheter, wherein the axial displacement is controlled by connecting at least one of the stents to a restraining member in the delivery catheter. In this case, the selected number of stents becomes detached from the stents remaining in the delivery catheter upon deployment.

In a further arrangement, a stent delivery catheter includes an outer shaft forming a first lumen, a plurality of self-expanding tubular stents carried within the first lumen, and a movable coil member interactive with the stents to control expansion of the stents when the stents are deployed from the first lumen. The stents are generally adapted to radially expand upon deployment from the first lumen.

In some such arrangements, the coil member is removable from the deployed stents by rotating the coil member. In some such arrangements, the stents have sidewalls with a plurality of openings, the coil member being threaded through the openings. Alternatively, the stents may include a plurality of struts, at least one of the struts being bent inwardly, with the coil member being threaded through the inwardly bent struts. Optionally, the coil member may be radially expandable to allow controlled expansion of the stents. In some such arrangements, a distal portion of the coil member is retractable into the outer shaft following deployment of the selected number of stents. In some such arrangements, the stents are disposed within the coil member.

In various such arrangements, the coil member may include a plurality of loops forming a helix. For example, in some embodiments between 2 and 6 loops are disposed in each stent. In other arrangements, more than 6 loops are disposed in each stent. In some such arrangements, the coil member comprises a plurality of loops contacting each other to form a continuous tube.

Optionally, the delivery catheter may also include a deployment mechanism for deploying a selected number of stents from the inner lumen. In some such arrangements, for example, the deployment mechanism includes a pushing element slidably disposed in the first lumen, the pushing element being in engagement with at least one of the stents to advance the stents distally relative to the outer shaft. Optionally, adjacent ends of adjacent stents may be interleaved to resist rotation of the stents relative to each other. In one such arrangement, a distal end of the pushing element is interleaved with a proximal end of a proximal-most stent to resist rotation of the stents. In these or other arrangements, the coil member may optionally be configured to maintain rotational position of the stents relative to each other.

In another arrangement, a stent delivery catheter for delivering stents into a vessel lumen includes an outer shaft forming a first lumen, an inner shaft slidably disposed within the first lumen, an evertible tube having a first end coupled with a distal end of the outer shaft and a second end coupled with a distal end of the inner shaft, and a plurality of self-expanding tubular stents carried within the evertible tube. Again, the stents are generally adapted to radially expand upon deployment from the evertible tube. Moving the outer shaft proximally relative to the inner shaft everts a distal portion of the evertible tube so as to deploy one or more of the stents.

In some such arrangements, an inner surface of the inner shaft comprises at least one adherent element for releasably holding the stents to the inner surface. For example, in one arrangement, the adherent element comprises a tacky surface coating. Alternatively, the adherent element may comprise a softenable material into which the stents are removably embedded. In other arrangements, the adherent element comprises a plurality of inwardly-facing protrusions positioned to extend through openings in the stents. Such protrusions may have any of a number of shapes in various arrangements, such as but not limited to mushroom-shaped, L-shaped, T-shaped, hook-shaped, rounded, spiked, pyramidal, barbed, arrow-shaped or linear. In yet other such arrangements, the adherent element may comprise a structure such as but not limited to bumps, bristles, spines, ridges ribs, waves, grooves, pits, channels, detents or random surface irregularities.

In another aspect but outside the scope of the present invention, a method of delivering one or more stents to a treatment site in a vessel involves: positioning a delivery catheter at the treatment site, the delivery catheter carrying a plurality of self-expanding stents; selecting a desired number of the stents to deploy; deploying the desired number of stents from the delivery catheter into the vessel, each stent expanding into contact with the vessel upon deployment; controlling axial displacement of each of the selected number of stents relative to the delivery catheter during deployment of the pstents with an expandable coil member coupled with the stents; and removing the expandable coil member from the deployed stents.

In some arrangements, removing the coil member involves rotating the coil member. For example, the coil member may be helically threaded through the stents such that rotating the coil member unthreads the coil member from one or more stents. In some arrangements outside the scope of the present invention, the method also involves controlling the rotational displacement of the selected number of stents relative to the delivery catheter during deployment of the stents. In one such arrangement, for example, the rotational displacement is controlled by interleaving adjacent ends of adjacent stents and interleaving a proximal end of a proximal-most stent with a portion of the catheter device. In some such arrangements , a distal portion of the coil member expands with the selected number of stents.

In yet another aspect but outside the scope of the present invention, a method of delivering one or more stents to a treatment site in a vessel involves: positioning a delivery catheter at the treatment site, the delivery catheter carrying a plurality of self-expanding stents within an evertible tube; selecting a desired number of the stents to deploy; and everting a distal portion of the evertible tube to deploy the desired number of stents from the delivery catheter into the vessel, each stent expanding into contact with the vessel upon deployment. In some such arrangements, the distal portion of the evertible tube is everted by sliding an outer shaft of the catheter device relative to an inner shaft of the catheter device. For example, in some such arrangements, a distal end of the outer shaft is coupled with a distal end of the evertible tube such that sliding the outer shaft proximally relative to the inner shaft causes the distal end of the evertible tube to bend outward and fold over on itself.

Optionally, such a method may further involve controlling axial displacement of each of the selected number of stents relative to the delivery catheter during deployment of the stents by contacting an adherent inner surface of the evertible tube with the stents. In one arrangement, for example, the adherent surface maintains engagement with the stents until the distal portion of the evertible tube is peeled away from the stents. In some arrangements, the adherent surface comprises a friction- inducing coating or friction-inducing surface feature. In some arrangements, contacting the adherent surface with the stents involves releasably coupling one or more retention structures on the inner surface with the stents. Alternatively, contacting the adherent surface with the stents may involve embedding the stents in a deformable material on the adherent inner surface.

In a further illustrative arrangement, a stent delivery catheter comprises an outer shaft having a distal end and a first lumen, a plurality of self-expanding tubular stents carried within the first lumen, the stents being adapted to radially expand upon deployment from the first lumen, and a control member extending distally from the distal end of the outer shaft and defining an interior communicating with the first lumen for receiving one or more of the stents. The control member has an undeflected shape when not engaged by one of the stents and is configured to deflect radially outwardly when engaged by a stent during expansion thereof. The control member is also configured to resiliently return to the undeflected shape when the stent is removed from the interior. In one such arrangement, the control member generally includes a plurality of deflectable tines having free distal ends received within an aperture on the nose cone or nose piece of the catheter. Optionally, the control member may further include a plurality of webs between the tines. In an alternative arrangement, the control member may comprise a distensible tubular structure.

Further aspects of the nature and advantages of the invention will be apparent from the following detailed description of various embodiments and illustrative arrangements of the invention taken in conjunction with the drawings, subject to the provisothat methods of use of the stent delivery catheter are also beyond the scope of the presently claimed invention, although retained as helpful to the reader to gain a better appreciation of the present invention and its function.

### BRIEF DESCRIPTION OF THE DRAWINGS

Fig. 1 is a side cut-away view of a stent delivery catheter according to the invention,
Fig. 2A is a side cross-sectional view of a distal portion of a stent delivery catheter according to the invention in a further embodiment thereof,
Fig. 2B is a side cross-sectional view of the stent delivery catheter of Fig. 2A showing the deployment of stents in a vessel,
Figs. 3A-3C are perspective, side, and end views respectively of a stent coupled to control wires according to further embodiments of the invention,
Fig. 4A is a side cross-section of a distal portion of a stent delivery catheter according to an illustrative arrangement.
Fig. 4B is a side cross-section of the stent delivery catheter of Fig. 4A showing the deployment of stents in a vessel,
Fig. 5A is a side cross-section of a distal portion of another arrangement of a stent delivery catheter.
Fig. 5B is an oblique view of a distal portion of a stent delivery catheter according to yet another illustrative arrangement.
Figs. 6A-6C are side cross-sectional views of a distal portion of a stent delivery catheter according to still anotherarrangement, showing the outer shaft unretracted, outer shaft retracted with sleeve unexpanded, and sleeve with stents expanded, respectively,
Figs. 7A-7B are side cross-sectional views of a distal portion of a stent delivery catheter according to another arrangement, showing outer shaft retracted with sleeve unexpanded, and outer shaft retracted with sleeve and stents expanded, respectively,
Figs. 8A-8C are side cross-sectional views of a distal portion of a stent delivery catheter according to the invention in a further arrangement, showing the outer shaft unretracted, outer shaft retracted with sleeve unexpanded, and sleeve with stents expanded, respectively,
Figs. 9A-9B are side cross-sectional views of a distal portion of a stent delivery catheter in a vessel in another arrangement, showing outer shaft retracted with stent partially deployed, and stent fully deployed, respectively,
Figs. 10A-10B are side cross-sectional views of a distal portion of a stent delivery catheter in a vessel in yet another arrangement, showing outer shaft retracted with stent partially deployed, and stent fully deployed, respectively,
Figs. 11A-11C are side cross-sectional views of a distal portion of a stent delivery catheter in a vessel in yet another arrangement, showing a first stent deployed, an expandable member expanded within the first stent, and a second stent deployed with expandable member expanded in the first stent, respectively,
Figs. 11D-11F are side cross-sectional views of a distal portion of a stent delivery catheter in another arrangement, showing the delivery catheter prior to stent deployment, the deployment of a first stent in a vessel, and a deployed stent in the vessel, respectively,and
Fig. 12 is a side cross-sectional view of a distal portion of a stent delivery catheter in a vessel in still another arrangement, showing a first stent deployed in a lesion.

### DETAILED DESCRIPTION OF THE INVENTION

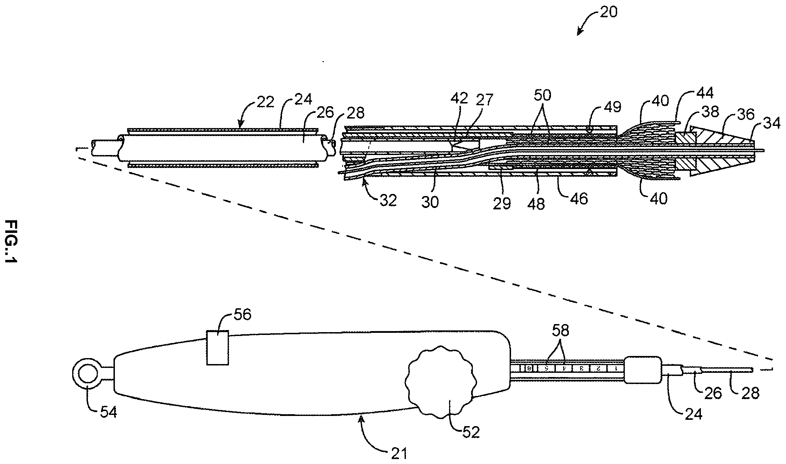

Referring to Figure 1, a first embodiment of a stent delivery catheter according to the invention is illustrated. Delivery catheter 20 may have any of various constructions, including that described in co-pending application Serial No. 10/637,713, filed August 8, 2003. (US2004/215,312). Delivery catheter 20 has a handle assembly 21 and an elongated catheter body 22 that includes three concentric tubular shafts all axially slidable relative to one another: an outer shaft 24, a pusher 26, and an inner shaft 28. Pusher 26 has a distal extension 27 to which a pusher ring 29 is fixed. In a distal region of the catheter body 22, a guidewire tube 30 extends slidably through a port 32 in outer shaft 24 and through pusher ring 29 and has a distal end 34, to which is mounted a nosecone 36 and a stop member 38.

Delivery catheter 20 further includes one or more stent expansion control members, which in the illustrated embodiment comprise a plurality of control wires 40. Preferably, one or more pairs of control wires 40 are mounted on opposing sides of delivery catheter 20, e.g. four control wires 40 offset 90° from each other. Control wires 40 are fixed at their proximal ends 42 to inner shaft 28, and have free distal ends 44.

Outer shaft 24 has a distal extremity 46 defining a first lumen 48. A plurality of stents 50 are disposed in a collapsed configuration within first lumen 48. Stents 50 are preferably composed of a resilient material such as stainless steel or Nitinol so as to self-expand from the collapsed configuration to a radially expanded configuration when deployed from first lumen 48. While stents 50 as illustrated have a wave-like or undulating pattern in a plurality of interconnected circumferential members, the pattern illustrated is merely exemplary and the stents of the invention may have any of a variety of strut shapes, patterns, and geometries. From 2 up to 10 or more stents may be carried by outer shaft 24. Optionally, a valve member 49 is mounted within first lumen 48 to facilitate separating those stents 50 to be deployed from those to remain within outer shaft 24, as described in co-pending application Serial No. 10/412,714, filed April 10, 2003, (US2004/098,081).

Control wires 40 run along the outside of stents 50 or through the interior of stents 50, are threaded through openings in the walls of stents 50 or are otherwise coupled with stents 50 to control the deployment thereof, as described more fully below. Control wires 40 are composed of a resilient material such as stainless steel, Nitinol, or a suitable polymer, and are preferably generally straight and biased inwardly against guidewire tube 32 or to a position generally parallel to the axial direction. In Fig. 1, outer shaft 24 has been retracted to expose a plurality of stents 50 which are partially expanded and remain coupled to or restrained by control wires 40, as explained in greater detail below.

Handle assembly 21 has a rotatable retraction knob 52 coupled to a shaft housing 53, to which outer shaft 24 is fixed. By rotating retraction knob 52, outer shaft 24 may be retracted proximally relative to pusher 26 and inner shaft 28. A pull ring 54 is coupled to inner shaft 28, allowing inner shaft 28, and hence control wires 40, to be retracted proximally relative to outer shaft 24. A switch 56 engages and disengages pusher 26 with outer shaft 28, so that pusher 26 either moves with outer shaft 24 or remains stationary as outer shaft 24 is retracted. Indicia 58 on shaft housing 53 indicate the extent of retraction of outer shaft 28 by distance, number of stents, or other suitable measure. Other aspects of handle assembly 21 are described in co-pending application Serial No. 10/746,466 filed December 23, 2003 (US2007/156 225).

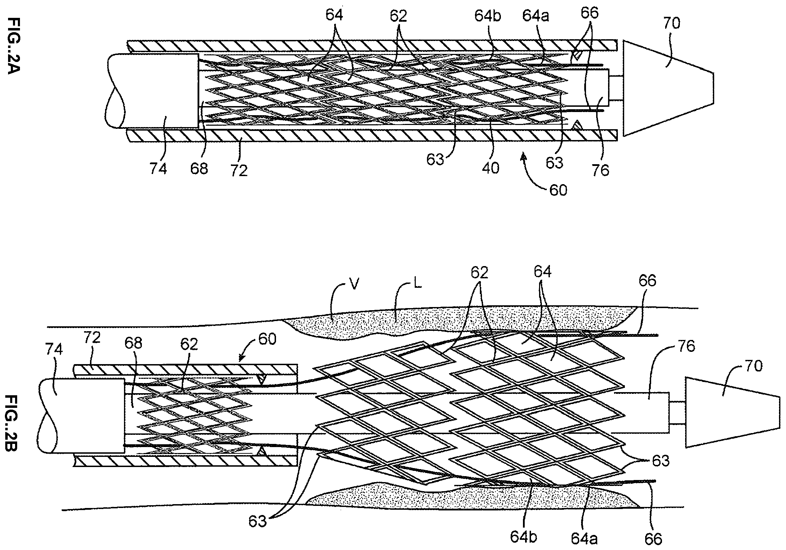

Figures 2A-2B illustrate a distal extremity of a stent delivery catheter 60 according to the invention in a further embodiment thereof. In this embodiment, stents 62 have a series of diamond shaped openings 64 in the walls thereof through which a plurality of control wires 66 are threaded. Stents 62 have a plurality of axially-extending V-shaped points 63 on their distal and proximal ends. These points 63 are configured to interleave or nest with the points 63 on the adjacent stent 62, preferably both in the collapsed and expanded configurations. Various suitable interleaving stent geometries are described in co-pending application Serial No. 10/738,666, filed December 16, 2003, (US2004/0186551) . In order to maintain this interleaving, it is important to maintain the relative rotational and axial positions of the adjacent stents 62 both before and during deployment. By extending through the openings 64 in each stent, control wires 66 keep adjacent stents 62 in rotational alignment as they are advanced forward through the catheter and during deployment. Preferably, each control wire 66 is threaded through at least two openings 64 in each stent 62, one opening 64a near the distal end of each stent 62 and one opening 64b near the proximal end of each stent 62. Alternatively, control wires 66 may be threaded through only a single opening 64 or through three or more openings 64 on each stent 62. Preferably, however, control wires 66 are threaded so that the distal and proximal ends of stents 62will expand at a generally uniform rate when released, as described below.

Control wires 66 are constructed of a resilient and flexible metal or polymer with sufficient stiffness to provide controlled resistance to the expansion of stents 62. This stiffness may be selected to allow the desired expansion behavior of stents 62 such that "watermelon seeding" is avoided, inter-stent spacing is maintained, and sufficient stent expansion occurs. Control wires 66 may have various cross-sectional geometries, and may be flat ribbons or blades, round or oval wires, I-beams, or other suitable structures to control stent expansion, maintain spacing and rotational position, and facilitate withdrawal from stents 62 without interference. Control wires 66 may be composed of or coated with a lubricious material such as PTFE to reduce friction during removal from stents 62. In other embodiments, control wires 66 may have surface features, be wrapped with wire windings, or be coated with "sticky" material to increase friction with stents 62. Coatings or surface structures such as scales with one-way frictional effects may also be applied to control wires 66.

As a further alternative, control wires 66 may comprise flexible hollow tubes which are pneumatically or hydraulically controllable to vary their rigidity or stiffness. For example, control wires 66 may comprise polymeric tubes that radially contract or flatten and are very flexible when evacuated of fluid, but which become more rigid when filled with pressurized fluid, such as saline, air, or other liquid or gas. In such an embodiment, control wires 66 are fluidly connected to a pump, syringe, or other suitable fluid delivery mechanism at the proximal end of the delivery catheter. In this way, control wires 66 may be pressurized to increase stiffness as stents 62 are deployed, then evacuated of fluid to reduce their profile and stiffness during withdrawal from the deployed stents:

Stents 62 are slidably positioned over an inner shaft 68, to which is attached a nosecone 70 at the distal end of the device. An outer shaft 72 is slidably disposed over inner shaft 68 and surrounds stents 62, maintaining them in a collapsed configuration, as shown in Fig. 2A. A pusher shaft 74 is slidably disposed over inner shaft 68 and is configured to engage the proximal end of the proximal-most stent 62. Outer shaft 72 is retractable relative to inner shaft 68 in order to expose a desired number of stents 62 as shown in Fig. 2B. When outer shaft 72 is retracted, the exposed stents 62 self-expand to a larger-diameter expanded shape in engagement with lesion L in vessel V. Preferably, at least the distal end of the distal-most stent 62, and more preferably a substantial portion of all stents 62 being deployed, is allowed to expand into engagement with lesion L while control wires 66 remain threaded through openings 64. Control wires 66 are then withdrawn from openings64, preferably by holding catheter 60 in position and pulling control wires 66 proximally using a suitable mechanism such as that described above with reference to Fig. 1. Alternatively, the entire catheter 60 may be retracted proximally relative to stents 62 to withdraw control wires 66 from openings 64. Because at least a portion of stents 62 is in engagement with lesion L, stents 62 are held in position in the vessel as control wires 66 are withdrawn.

Optionally, inner shaft 68 may have a balloon 76 mounted thereto near its distal end to enable pre- or post-dilatation of lesion L. In this embodiment, inner shaft 68 has an inflation lumen through which inflation fluid may be delivered to balloon 76. Balloon 76 is preferably as long as the longest lesion that might be treated using catheter 60. To dilate lesion L prior to stent deployment, or to further expand stents 62 after deployment, outer shaft 72 and those of stents 62 remaining therein are retracted relative to inner shaft 68 to expose a desired length of balloon 76. The exposed portion of balloon 76 may then be inflated within the lesion L and/or the deployed stents 62.

Following deployment and any post-dilatation, inner shaft 68 is retracted into outer shaft 72 while maintaining pressure against pusher shaft 74. This slides stents 62 distally along control wires 66 and repositions stents 62 to the distal end of inner shaft 68 so as to be ready for deployment. Catheter 60 may then be repositioned to another vascular location for deployment of additional stents 62.

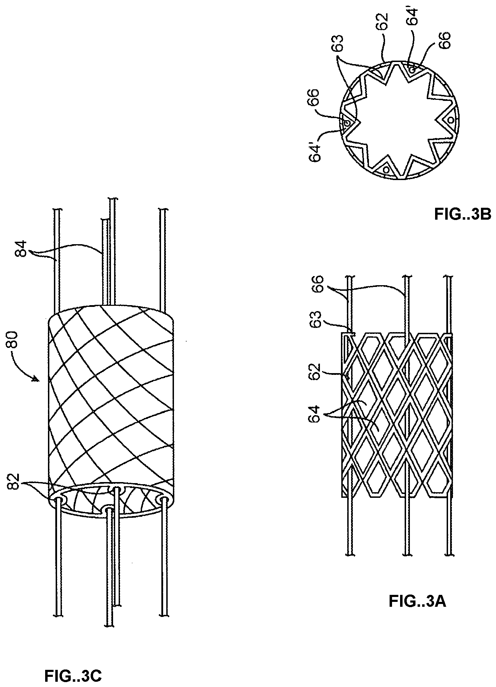

Control wires 66 may be coupled to stents 62 in various ways, some of which depend upon the configuration of stents 62. For example, as shown in Figs. 3A-B, the points 63 at the ends of each stent 62 may be bent inwardly such that a portion of the openings 64' are oriented axially. Control wires 66 may then be threaded through these axially-oriented openings 64'. Preferably, upon deployment, points 63 are adapted to deform with stent expansion so as to be more parallel to the axial direction, thereby providing a smooth and open flow path through the stent.

In another embodiment, shown in Fig. 3C, stents 80 have axially-aligned eyelets 82 through which control wires 84 are threaded. These eyelets 82 may be in the interior of stents 82 as shown in Fig. 3C, or such eyelets may be on the exterior surface of stents 82, or could be drilled through one or more of the struts of stents 82. Various other structures may also be used for coupling the stents of the invention to control wires, including hooks, channels, holes, sleeves, and others, disposed on the interior, exterior or end surfaces of the stent, or through the struts themselves. Such structures may beintegral with stent struts and of the same material, may be attached to the stent struts and be of same or different material, or may be a biodegradable material that erodes and eventually is absorbed into the body following deployment.

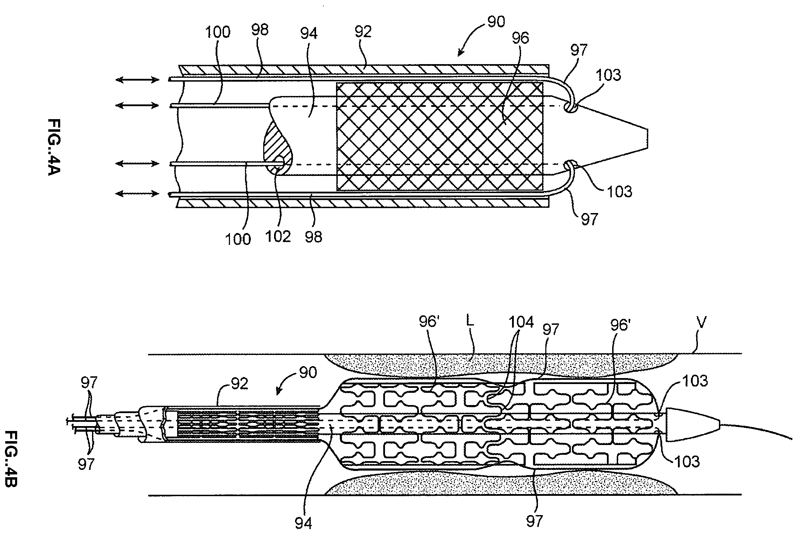

Referring now to Figures 4A-4B, in an illustrative arrangement, a stent delivery catheter 90 has an outer shaft 92 slidably disposed over an inner shaft 94, and at least one stent 96 (shown schematically in Fig. 4A) in a collapsed shape within outer shaft 92. A plurality of control wires 97 have an outer extremity 98 outside of inner shaft 94 and an inner extremity 100 extending through one or more lumens 102 and distal ports 103 in inner shaft 94. Both outer portion 98 and inner portion 100 extend proximally to the proximal end of delivery catheter 90. Outer extremities 98 are threaded through openings in the wall of stent 96 or are otherwise coupled thereto as described above so as to resist expansion of stent 96 upon deployment. Control wires 97 thus form a continuous loop from the proximal end of stent delivery catheter 90, through stent 96 and back to the proximal end of the catheter.

Fig. 4B illustrates this arrangement of delivery catheter 90 positioned in a vessel V and carrying plurality of stents 96'. Stents 96' have axial projections 104 at their distal and proximal ends configured to interleave when stents 96' are collapsed within outer shaft 92 and when deployed in vessel V. When outer shaft 92 is retracted to expose one or more stents 96', the expansion of stents 96' can be resisted and controlled by maintaining tension on control wires 97. Tension may be controllably relaxed to allow stents 96' to expand into contact with lesion L, as shown in Fig. 4B. By controlling the expansion in this way, the axial spacing and rotational positions of adjacent stents 96'may be maintained so that gaps and overlaps are minimized and the interleaving of axial projections 104 is maintained. When stents 96' are fully expanded, one end of each control wire 97 may be released at the proximal end of delivery catheter 90 while the other end is pulled to retract the control wires from stents 96'.

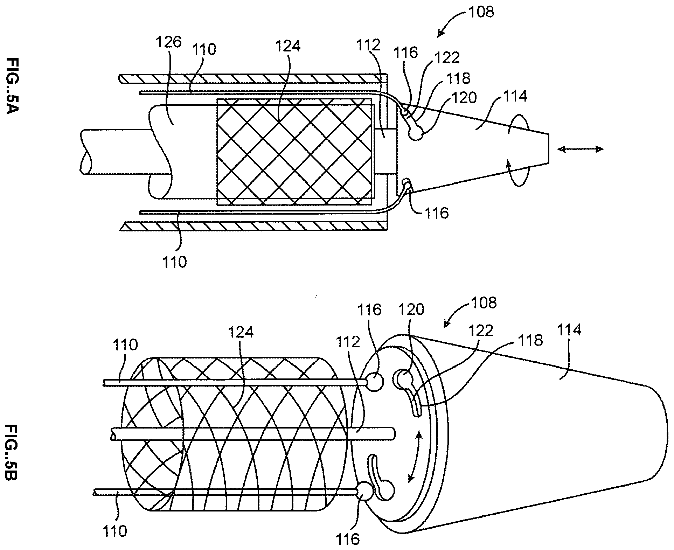

In a further arrangement, illustrated schematically in Figs. 5A-B, delivery catheter 108 is constructed as described above except that control wires 110 are releasably coupled to the distal end of an inner shaft 112 or to nose cone 114. In an exemplary arrangement, control wires 110 have balls 116 at their distal ends configured to be received within slots 118 on the outer surface of nosecone 114 (Fig. 5A) or on the proximal face of nosecone 114 (Fig. 5B; outer shaft not shown for clarity). Slots 118 have an enlarged portion 120 of sufficient size to receive ball 116 and a narrow portion 122 through which balls 116 may not pass. Inner shaft 112 is axially rotatable relative to control wires 110. As in the arrangement of Figs. 4A-B, with balls 116 held within slots 118, tension may be maintained on control wires 110 to resist expansion of stent 124. Stent 124 may be allowed to expand by gradually relaxing tension on control wires 110. Once stent 124 is fully expanded tension on control wires 110 may be fully relaxed and nosecone 114 then rotated by rotating inner shaft 112, thereby allowing balls 116 to pass through enlarged portions 120. Control wires 110 may then be withdrawn from the deployed stent 124. Nosecone 114 is then retracted or control wires 110 advanced so as to reinsert balls 116 into slots 118. Nosecone 114 is then rotated to align balls 116 with narrow portions 122, again securing the control wires to nosecone 114. Delivery catheter 108 may then be repositioned to deploy additional stents.

Optionally, delivery catheter 108 may include a middle shaft or balloon 126 over which stents 124 are positioned, as shown in Fig. SA. In this case, inner shaft 112 is slidably and rotatably disposed in an inner lumen though middle shaft or balloon 126. If a balloon is included, it may be used for pre-dilatation of lesions prior to stent deployment, or for further expansion of stent 124 following deployment.

In the foregoing arrangement, control wires 110 will be constructed to have sufficient stiffness to resist rotation, twisting or bending as nosecone 114 is rotated to release control wires 110. Maintaining some tension on control wires 110 as nosecone 114 is rotated may facilitate the release process. In addition, control wires 110 will have sufficient column strength to facilitate reinsertion into slots 118 following deployment of stents 124. Thus the size, material and geometry of control wires 110 will be selected to enable these actions while providing the desired level of control of stent expansion.

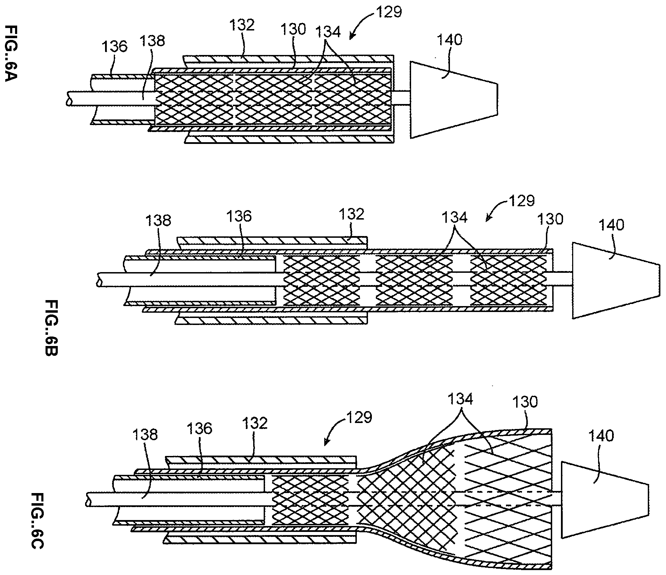

In a further arrangement of a stent delivery catheter an expandable sleeve 130 is slidably positioned within outer shaft 132 and carries stents 134 as shown in Figs. 6A-C. A pusher shaft 136 is slidable within sleeve 130 and engages the proximal-most stent 134. An inner shaft 138 extends through pusher shaft 136 and has a nosecone 140 fixed to its distal end. Sleeve 130, or at least a distal extremity thereof, may be a tube constructed of a resilient deformable material such as urethane or other medical grade elastomer, or may be a tubular mesh, cage, grating, or other suitable structure of flexible and resilient polymer or metal such as stainless steel or Nitinol. The elasticity and stiffness of sleeve 130 are selected to allow stents 134 to expand at the desired rate when deployed from outer shaft 132 without excessive axial or rotational displacement relative to each other or to outer shaft 132. Sleeve 130 is resiliently biased toward an unexpanded shape so that following stent deployment, sleeve 130 returns to a generally tubular shape. Outer shaft 132 is constructed of a material with sufficient radial strength and stiffness to resist expansion of stents 134 and sleeve 130, and may include a metallic or polymeric braid, ribs, rings or other structural reinforcement near its distal end for such purpose.

The interior surface of sleeve 130 optionally may have surface features such as bumps, scales, bristles, ribs, or roughness to enhance friction with stents 134. These features may be configured to have a grain such that they provide more friction against movement in the distal direction than in the proximal direction, or vice versa. Further, such features may be adapted to provide more friction when sleeve 130 is in an unexpanded shape than when it is expanded by stents 134. For example, bristles may be provided that point more in the proximal direction when sleeve 130 is in its unexpanded cylindrical shape, but which point more distally or radially (perpendicular to the surface of sleeve 130) when sleeve 130 is expanded. This allows sleeve 130 to be more easily withdrawn from stents 134 when stents 134 are deployed.

In order to deploy stents 134, delivery catheter 129 is positioned across a vascular lesion so that nosecone 140 is disposed just distally of the distal end of the lesion. Outer shaft 132 is then retracted to expose the desired number of stents 134 (and the associated length of sleeve 130) which will cover the length of the lesion, as shown in Fig. 6B. As outer shaft 132 is retracted, stents 134 are allowed to expand into contact with the lesion as shown in Fig. 6C. Sleeve 130 controls the rate of expansion and maintains the positions of stents 134 so they are deployed precisely at the intended location. Once stents 134 are fully expanded, sleeve 130 may be retracted from between the stents and the lesion until sleeve 130 is again disposed in outer shaft 132. Pressure is maintained on pusher shaft 136 during this process so that the stents 134 remaining in delivery catheter 129 are advanced to the distal end of sleeve 130 and outer shaft 132. Delivery catheter 129 may then be repositioned for deployment of additional stents at other locations.

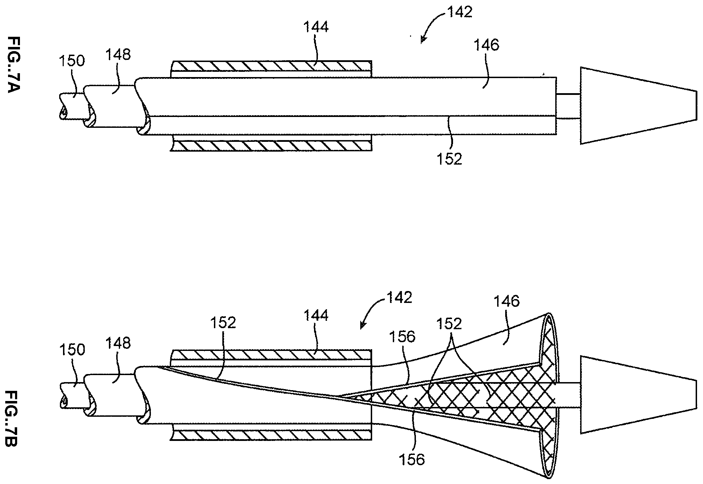

Referring now to Figs. 7A-B, in a further arrangement, a delivery catheter 142 may be constructed largely as described in connection with Figs. 6A-C, including an outer shaft 144, an expandable sleeve 146 slidably disposed therein, a pusher shaft 148, and inner shaft 150. A plurality of stents 152 are carried in expandable sleeve 146 (shown in Fig. 7B). In order to facilitate expansion, expandable sleeve 146 includes a longitudinal slit 154 in at least a distal extremity thereof When outer shaft 144 is retracted relative to sleeve 146, sleeve 146 may be controllably expanded by axially twisting sleeve 146 such that the opposing edges 156 along longitudinal slit 154 pivot away from one another, forming a cone shape (Fig. 7B). In this way, the expansion of stents 152 is further controllable after retraction of outer shaft 144 by controlling the rate of twisting of sleeve 146. An actuator may be provided at the proximal end of delivery catheter 142 to control such twisting. Optionally, sleeve 146 may have a helical thread on its outer surface that mates with a complementary thread on the interior of outer shaft 144 such that sleeve 146 is automatically twisted as outer shaft 144 is retracted. As in the arrangement of Figs. 6A-C, following stent deployment, sleeve 146 is retracted from the space between the deployed stents and the vessel wall and returned within outer shaft 144. Sleeve 146 may be resiliently biased to return to its unexpanded configuration, or may be manually twisted back to an unexpanded shape by the operator.

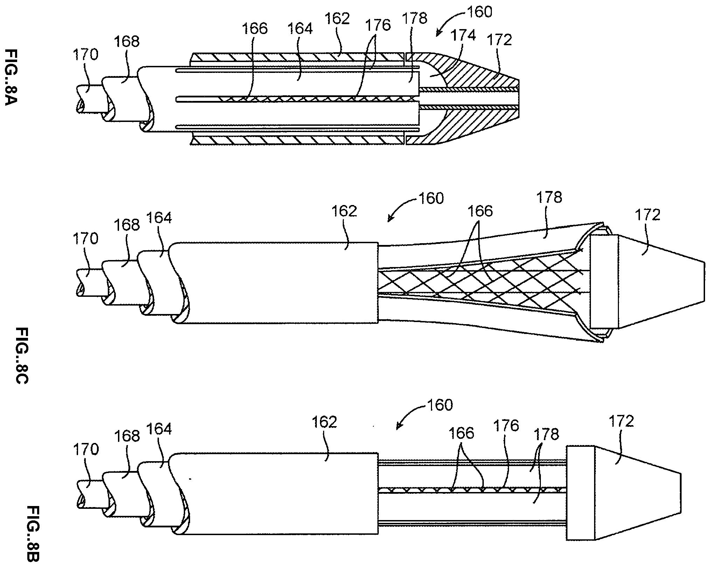

In another arrangement, shown in Figs. 8A-C, delivery catheter 160 is again constructed much like delivery catheter 129 of Figs. 6A-C, including an outer shaft 162, a slidable expandable sleeve 164 carrying stents 166, a pusher shaft 168, and an inner shaft 170. A nosecone 172 is attached to the distal end of inner shaft 170 and has a concavity 174 at its proximal end configured to receive the distal end of sleeve 164. A distal extremity of sleeve 164 includes a plurality of axial slits 176 defining separate deflectable longitudinal beams 178. Sleeve 164 includes at least two, preferably four, and as many as six, eight, or more slits 176 to provide a corresponding number of longitudinal beams 178. Longitudinal beams 178 are resiliently biased into an axial orientation wherein sleeve 164 is generally cylindrical. Longitudinal beams 178 have sufficient stiffness against lateral deflection to resist and control the expansion of stents 166.

Advantageously, by containing the distal ends oflongitudinal beams 178 in concavity 174, outer shaft 162 may be retracted to expose the desired number of stents to cover a target lesion without immediate expansion of stents 166, as shown in Fig. 8B. When the desired number of stents 166 is exposed, inner shaft 170 may be advanced distally relative to sleeve 164, releasing longitudinal beams 178 from concavity 174. This permits longitudinal beams 178 to laterally deflect, allowing stents 166 to expand, as shown in Fig. 8C. When full expansion is achieved, longitudinal beams 178 may be retracted from between stents 166 and the vessel wall. Longitudinal beams 178 then return to their undeflected axial orientation, allowing inner shaft 170 to be retracted so as to return the distal ends oflongitudinal beams 178 into concavity 174. Inner shaft 170 and sleeve 164 may then be retracted into outer shaft 162 while maintaining pressure on pusher shaft 168, thereby advancing additional stents 166 toward the distal end of sleeve 164 for additional deployments.

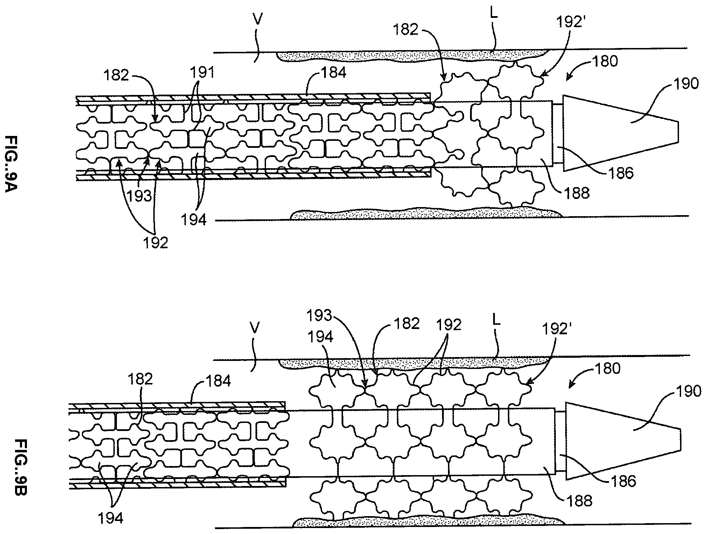

In some arrangements of the stent delivery catheter of the invention, the stents themselves are configured to provide greater control and precision in stent deployment. For example, Figs. 9A-9B illustrates a delivery catheter 180 having a plurality of stench. 182 disposed in an outer shaft 184. An inner shaft 186 with optional balloon 188 and nosecone 190 extends through outer shaft 184 and stents 182 and is axially movable relative thereto. A pusher shaft (not shown) is slidably disposed over inner shaft 186 and engages stents 182 for purposes of deploying stents 182 from outer shaft 186 and repositioning the remaining stents 182 within outer shaft 186, as in earlier arrangements. In this arrangement, stents 182 comprise a plurality of struts 191 forming a series of rings 192 interconnected at joints 193. Each ring 192 has a series of closed cells 194 interconnected circumferentially and having an "I" shape in the unexpanded configuration.

As outer shaft 184 is retracted to deploy one or more stents 182, at least a distal ring 192' is configured to expand into engagement with the vessel wall before the entire length of the stent 182 is deployed from outer shaft 184 (Fig. 9A). Once in engagement with the lesion Lin vessel V, distal ring 192' anchors stent 182 in position as the remainder of the stent is deployed (Fig. 9B), preventing "watermelon seeding" of the stent from the catheter. The axial length of stent 182, the length of each ring 192, the number of rings, the stiffness of struts 191, and the flexibility of joints 193 are all selected to optimize this deployment behavior. Each stent 182 has at least two, and preferably four or more rings 192, each ring being about 2-5 mm in length, giving stent 182 an overall length of at least about 8-20 mm. Of course, stents of shorter or longer length are also contemplated . Lesions longer than each stent 182 may be treated by deploying multiple stents 182 end-to-end. Advantageously, each stent 182 can be deployed precisely at a desired spacing from a previously-deployed stent 182 because the distal ring 192' of each stent 182 can be first allowed to expand into engagement with the vessel at the target location, anchoring the stent in position as the remainder is deployed.

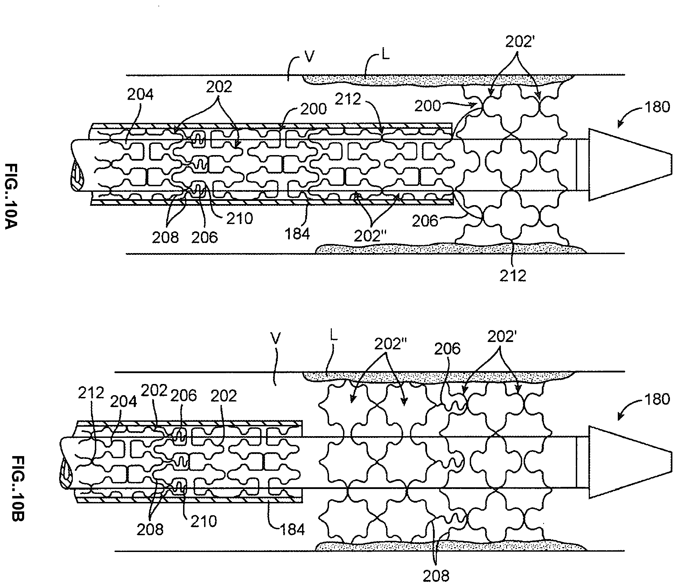

Rings 192 are preferably formed from a common piece of material and are integrally interconnected at joints 193, making joints 193 relatively rigid. In this arrangement,, the majority of flexibility between rings 192 is provided by struts 191 rather than by joints 193. Alternatively, joints 193 may comprise welded connections between rings 192 which are also fairly rigid. As a further alternative, joints 193 may comprise hinge or spring structures to allow greater deflection between adjacent rings 192, as exemplified in Figs. 10A-10B, described below.

In the arrangement of Fig. 10A-10B, stents 200 are constructed similarly to stents 182 of Figs. 9A-98, including a plurality of interconnected rings 202 having I-shaped cells 204. However, in this arrangement, some of rings 202 are interconnected by spring members 206 that may be elongated to increase the distance between rings 202 and that are resiliently biased into a shortened configuration to draw rings 202 toward each other. In one arrangement, spring members 206 have a wave-like shape and extend from the tip of an axial projection 208 on one ring 202 to a concave portion 210 between axial projections 208 on the adjacent ring 202. Of course a variety of spring configurations and connection locations are possible, including zig-zags, coils, spirals, accordian or telescoping structures, and the like. Further, resilient elongatable elastomeric elements may link the adjacent rings 202. In the illustrated arrangement, stent 200 comprises two pairs of rings 202, with the rings of each pair interconnected by integral joints 212 as in Figs. 9A-B and the pairs of rings 202 being connected to each other by spring members 206. Stents 200 may alternatively include two, three, five, six or more rings 202, and spring members 206 may interconnect all or only a portion of rings 202.

Spring members 206 may be formed of the same or different material as that of rings 202, depending upon the desired performance characteristics. In addition, spring members 206 may be biodegradable so as to erode and eventually disappear, leaving the adjacent pairs of rings 202 unconnected.

During deployment, as outer shaft 184 is retracted to expose a stent 200, the distal pair of rings 202' first expands into engagement with lesion L in vessel V (Fig. 10A). Spring members 206 elongate to allow rings 202' to fully expand without pulling the second pair of rings 202" from outer shaft 184. As retraction of outer shaft 184 continues, the second pair of rings 202" expands and simultaneously is drawn toward distal ring pair 182' by contraction of spring members 206 (Fig. 10B). This results in a predictable and constant axial spacing between the adjacent pairs of rings 202. In addition, spring members 206 maintain rotational alignment of rings 202 to maintain the interleaving of axial projections 208 without overlap. As in previous arrangements, multiple stents 200 may be deployed sequentially from delivery catheter 180 to cover longer lesions. The ability to precisely deploy each stent permits the relative axial spacing and rotational position of such stents to be controlled to avoid excessive space or overlap.

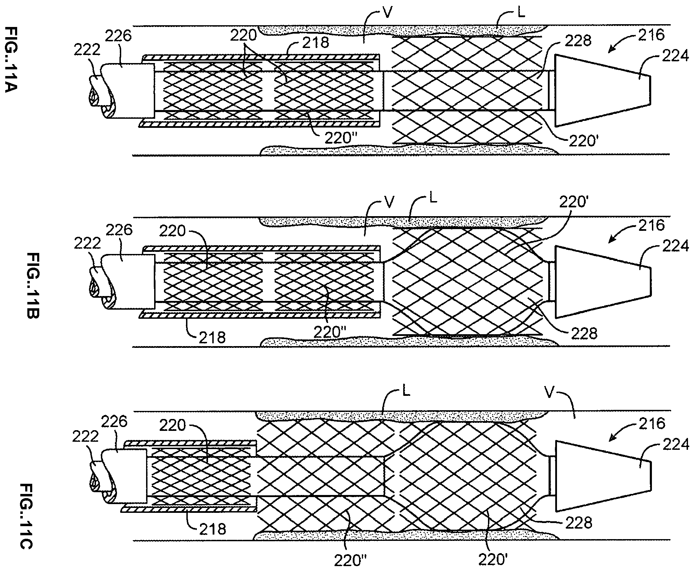

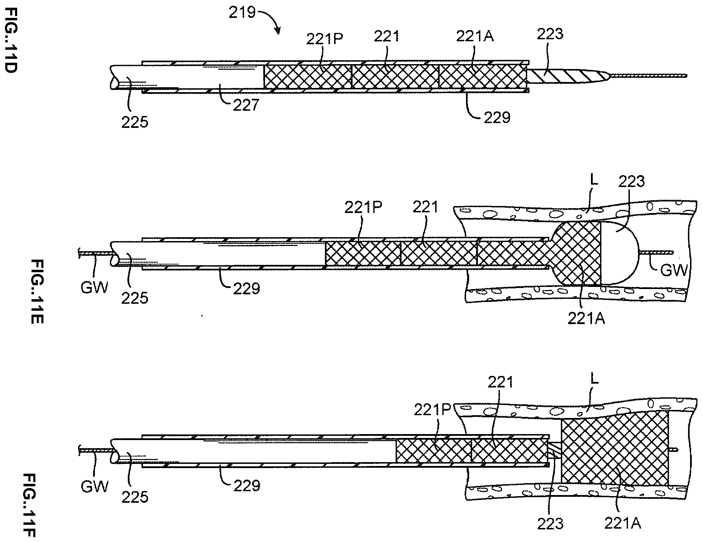

In a further arrangement, shown schematically in Figs. 11A-11C, a delivery catheter 216 has an outer shaft 218 carrying a plurality of stents 220. An inner shaft 222 extends through outer shaft 218 to a nosecone 224, and a pusher shaft 226 is slidably disposed over inner shaft 222. An anchoring balloon 228 is mounted to inner shaft 222 proximal to nosecone 224. Anchoring balloon 228 has an axial length sufficient to frictionally engage the wall of vessel V and remain stable so as to anchor delivery catheter 216 in place as further described below. Preferably, anchoring balloon 228 has a length about equal to the length of one of stents 220.

In use, outer shaft 218 is retracted so that a first stent 220' is released therefrom and expands into engagement with lesion L (Fig. 11A). Anchoring balloon 228 is then inflated until it engages the interior of stent 220' (Fig. 11B). This not only stabilizes delivery catheter 216, but may be used to further expand stent 220' and/or dilate lesion L to firmly implant stent 220'. While keeping anchoring balloon inflated within stent 220', outer shaft 218 is further retracted to release a second stent 220", which expands into engagement with lesion L (Fig. 11C). Advantageously, anchoring balloon 228 stabilizes delivery catheter 216 and anchors it in position relative to first stent 220' as second stent 220" is deployed. Second stent 220" is thus deployable precisely at the intended spacing and rotational position relative to first stent 220'. Anchoring balloon 228 may then be deflated and retracted into outer shaft 218, with pressure maintained upon pusher shaft 226 to reposition remaining stents 220 at the distal end of inner shaft 222.

Figs. 11D-11F illustrate another arrangement of a delivery catheter 219 in which a plurality of self-expanding stents 221 are slidably disposed over an elongated balloon 223. Balloon 223 preferably has a length as long as the longest lesion that is to be treated with the device, e.g. 50-200 mm. A pusher 225 is slidable relative to balloon 223 and has a tubular distal portion 227 disposed over balloon 223 which engages the proximal-most stent 221P. A sheath 229 is slidably disposed over pusher 225, stents 221 and balloon 223 and maintains stents 221 in a radially contracted configuration. In this arrangement, moderate pressure is maintained within balloon 223 during deployment of stents 221 so that the balloon expands simultaneously with each stent. As shown in Fig. 11B, as sheath 229 is retracted, a first stent 221A and a distal portion ofballoon 223 are exposed. By maintaining moderate inflation pressure in balloon 223 as sheath 220 is retracted the exposed portion ofballoon 223 expands with the first stent 221A, inhibiting distal migration of the stent from delivery catheter 219. One or more additional stents 221 maybe deployed by further retraction of sheath 229, during which balloon 223 remains expanded within first stent 221 anchoring the delivery catheter 219 in position (not shown). As each additional stent is exposed from sheath 229, the pressure in balloon 223 causes it to expand with the stent so as to control its rotational and axial position. Of course, stents 221 may have any of a variety of different configurations, including having open or closed cells, zig-zag or wave-shaped struts, and/or axially interleaving elements as described above.

Optionally, balloon 223 may have surface features or coatings on its periphery that enhance retention of stents 221 thereon. Such features may include structures such as scales or protuberances that are activated by pressurization of the balloon so that retention is lessened when the balloon is deflated, but heightened when the balloon is pressurized. Following stent deployment, pressure can optionally be increased in balloon 223 for post-dilation of stents 221. and the target lesion L. Balloon 223 is then deflated and retracted within sheath 229 as distal pressure is maintained against pusher 225, repositioning stents 221 near the distal end of balloon 223 within sheath 229 for deployment at another location, as shown in Fig. 11C.

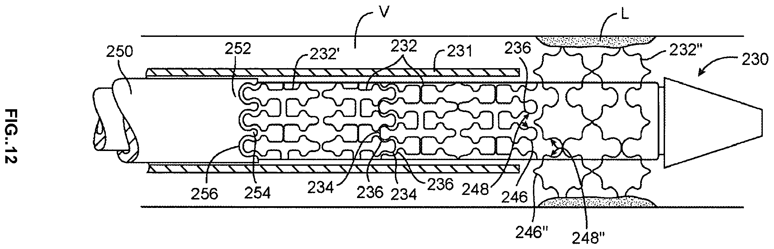

In a further arrangement, the stents in the delivery catheter may releasably interconnect with one another and/or with the pusher shaft to enable greater control and precision during deployment. As illustrated in Fig. 12, delivery catheter 230 carries a plurality of stents 232 having a structure much like that described above in connection with Figs. 9A-9B. However, in this arrangement, the axial projections 234 extending distally and proximally from stents 232 are configured to interconnect with concavities 236 on adjacent stents 232 until expanded. In one arrangement, axial projections 234 have enlarged heads 246 and concavities 236 have necks 248 that retain heads 246 within concavities 236 in the unexpanded configuration. Pusher shaft 250 has a distal end 252 having projections 254 and concavities 256 like those of stents 232, thus being able to interconnect with the proximal-most stent 232'. When a stent 232" expands, the interconnecting structures thereon are configured to separate from the adjacent stentor pusher shaft, thus releasing the deployed stent 232" from delivery catheter 230. In the example shown, as stent 232" expands, heads 246" contract in size while necks 248" enlarge, thereby allowing heads 246" on the expanded stent to be released from concavities 236 in the adjacent unexpanded stent, and vice versa. By exerting traction on pusher shaft 250 during the deployment process, the line of stents 232 is kept from moving distally relative to outer shaft 231, thus preventing the deployed stent 232" from "watermelon seeding" as it expands.

Various types of interconnecting structures between adjacent stents and between the stents and the pusher shaft are possible including those described in co-pending application Serial No. 10/738,666, filed December 16, 2003 (US 2004/0186551). Such interconnecting structures may also be breakable or frangible to facilitate separation as the stent expands. In addition, a mechanism such as an expandable balloon or cutting device may be disposed at the distal end of delivery catheter 230 to assist in separating stents 232 upon deployment. Further, the interconnections between stents may be different than the interconnection between the proximal-most stent and the pusher shaft. For example, the pusher shaft may have hooks, magnets, or other mechanisms suitable for releasably holding and maintaining traction on the proximal end of a stent until it is deployed.

## Claims

1. A stent delivery catheter (20,60)comprising: an outer shaft (24,72) having a first lumen (48) a plurality of self-expanding tubular stents (50,62)carried within the first lumen (48), the stents being adapted to radially expand upon deployment from the first lumen; and a deployment mechanism (26, 27, 29) for deploying a selected number of the stents from the first lumen (48)
**characterised in that** the stent delivery catheter further comprises a control member interactive with the stents to control expansion of the stents when the stents are deployed from the first lumen (48) the control member comprising a plurality of axially-extending control wires (40,66) the stents (50) being coupled to the wires and axially slidable thereon, and the wires being radially deflectable to allow controlled expansion of the stents, the stents having openings (50, 62) in the walls thereof through which the plurality of control wires are threaded,
and by extending through openings in each stent said wires keeping adjacent stents in rotational alignment when advanced forward.

2. A catheter (20,60) as claimed in claim 1, in which the stents have diamond shaped openings (64,64a,64b) in the walls thereof and a plurality of axially extending V-shaped points (63) on their distal and proximal ends.

3. A catheter (20,60) as claimed in claim 2, in which said points (63) are configured to interleave or nest with the points on the adjacent stents both in collapsed and expanded configurations.

4. A catheter (20,60)as claimed in claim 3, in which the interleaving is maintained by maintaining relative rotational and axial positions of adjacent stents (50,62,80,96,124,134,152,166,182,200,220,221,232)

5. A catheter (20,60) as claimed in any preceding claim, wherein the said control wires (40,66)are threaded through the openings in said stents (50,62)such that distal and proximal ends of stents can expand at a uniform rate upon release from the catheter.

6. A catheter (20,60)as claimed in any preceding claim, wherein said control wires (40,66)are constructed of a resilient and flexible metal or polymer with sufficient stiffness to provide controlled resistance to the expansion of stents (50,62)and have suitable structure to control stents expansion, to maintain stent spacing and rotational position and to facilitate withdrawal from the stents without interference.

7. A catheter (20,60)as claimed in any preceding claim in which the control wires (40,66)comprise flexible hollow tubes which are pneumatically or hydraulically controllable to vary their rigidity or stiffness.

8. A catheter (20,60)as claimed in any preceding claim comprising an inner shaft (28,68) to which a nosecone (36,70)is attached at the distal end of the device.

## Patentansprüche

1. Ein Stentzuführungskatheter (20, 60), umfassend: einen äußeren Schaft (24, 72) mit einem ersten Lumen (48), eine Vielzahl von selbstausdehnenden schlauchartigen Stents (50, 62), die im ersten Lumen (48) aufgenommen werden, die Stents sind so angepasst, dass sie sich beim Einsetzen vom ersten Lumen radial ausdehnen; und einen Einsetzmechanismus (26, 27, 29) für das Einsetzen einer ausgewählten Anzahl von Stents von dem ersten Lumen (48)
**gekennzeichnet dadurch, dass** der Stentzuführungskatheter ferner ein Steuerelement umfasst, das in Wechselwirkung zu den Stents steht, um die Ausdehnung der Stents zu steuern, wenn die Stents von dem ersten Lumen (48) eingesetzt werden, das Steuerelement umfasst eine Vielzahl von sich axial ausdehnenden Kontrolldrähten (40, 66), die Stents (50) sind mit den Drähten gekoppelt und darauf axial verschiebbar, und die Drähte sind radial biegbar, um eine kontrollierte Ausdehnung der Stents zu erlauben, die Stents verfügen über Öffnungen (50, 62) in ihren Wänden, in die die Vielzahl der Kontrolldrähte eingezogen sind, und dadurch, dass sie durch die Öffnungen in jedem Stent laufen, halten die Drähte benachbarte Stents in Rotationsausrichtung, wenn sie nach vorne verschoben werden.

2. Ein Katheter (20, 60) nach Anspruch 1, in dem die Stents diamantförmige Öffnungen (64, 64a, 64b) in ihren Wänden haben und eine Vielzahl von sich axial erstreckenden V-förmigen Punkten (63) auf ihren distalen und proximalen Enden aufweisen.

3. Ein Katheter (20, 60) nach Anspruch 2, in dem die besagten Punkte (63) konfiguriert sind, sich mit den Punkten an den benachbarten Stents, sowohl in der eingefalteten als auch der ausgedehnten Konfiguration, zu verschachteln oder ineinander zu stecken.

4. Ein Katheter (20, 60) nach Anspruch 3, in dem die Verschachtelung durch die Aufrechterhaltung relativer Rotations- und Axialpositionen der benachbarten Stents (50, 62, 80, 96, 124, 134, 152, 166, 182, 200, 220, 221, 232) beibehalten wird.

5. Ein Katheter (20, 60) nach einem der vorhergehenden Ansprüche, wobei die besagten Kontrolldrähte (40, 66) durch die Öffnungen in den Stents (50, 62) gezogen werden, so dass sich das distale und proximale Ende der Stents in gleichmäßiger Rate bei Freigabe aus dem Katheter ausdehnen.

6. Ein Katheter (20, 60) nach einem der vorhergehenden Ansprüche, wobei die besagten Kontrolldrähte (40, 66) aus einem elastischen und flexiblen Metall oder Polymer mit ausreichender Steifigkeit hergestellt sind, um der Ausdehnung der Stents (50, 62) einen kontrollierten Widerstand bieten zu können, und eine geeignete Struktur aufweisen, um die Stentausdehnung zu steuern, den Stent-Zwischenraum und die Rotationsposition beizubehalten und das ungestörte Herausziehen aus den Stents zu erlauben.

7. Ein Katheter (20, 60) nach einem der vorhergehenden Ansprüche, in dem die Kontrolldrähte (40, 66) flexible Hohlröhren umfassen, die pneumatisch oder hydraulisch steuerbar sind, um deren Festigkeit oder Steifigkeit zu variieren.

8. Ein Katheter (20, 60) nach einem der vorhergehenden Ansprüche, umfassend einen inneren Schaft (28, 68), an dem ein Nasenkonus (36, 70) am distalen Ende des Geräts angebracht ist.

## Revendications

1. Cathéter de pose d'endoprothèse (20, 60) comprenant : une tige extérieure (24, 72) présentant une première lumière (48) d'une pluralité d'endoprothèses tubulaires autodéployables (50, 62) transportées à l'intérieur de la première lumière (48), les endoprothèses étant aptes à se déployer radialement lors de la mise en place à partir de la première lumière ; et un mécanisme de mise en place (26, 27, 29) destiné à mettre en place un nombre sélectionné d'endoprothèses à partir de la première lumière (48)
**caractérisé en ce que** le cathéter de pose d'endoprothèse comprend en outre un élément de commande interactif avec les endoprothèses pour commander le déploiement des endoprothèses lorsque les endoprothèses sont mises en place à partir de la première lumière (48) l'élément de commande comprenant une pluralité de fils de commande s'étendant axialement (40, 66) les endoprothèses (50) étant couplées aux fils et pouvant glisser axialement dans celles-ci et les fils pouvant être déviés radialement pour permettre le déploiement commandé des endoprothèses, les endoprothèses présentant des ouvertures (50, 62) dans les parois correspondantes à travers lesquelles la pluralité de fils de commande sont enfilés et en s'étendant à travers les ouvertures de chaque endoprothèse, lesdits fils gardant les endoprothèses adjacentes en alignement par rotation lorsqu'elles vont vers l'avant.

2. Cathéter (20, 60) selon la revendication 1, dans lequel les endoprothèses présentent des ouvertures en forme de losange (64, 64a, 64b) dans les parois correspondantes et une pluralité de points en forme de V s'étendant axialement (63) sur leurs extrémités distales et proximales.

3. Cathéter (20, 60) selon la revendication 2, dans lequel lesdits points (63) sont conçus pour s'entrelacer ou se loger avec les points sur les endoprothèses adjacentes à la fois dans des configurations pliées et déployées.

4. Cathéter (20, 60) selon la revendication 3, dans lequel l'entrelacement est maintenu par le maintien des positions en rotation et axiales relatives des endoprothèses adjacentes (50, 62, 80, 96, 124, 134, 152, 166, 182, 200, 220, 221, 232)

5. Cathéter (20, 60) selon l'une quelconque des revendications précédentes, dans lequel lesdits fils de commande (40, 66) sont enfilés à travers les ouvertures dans lesdites endoprothèses (50, 62) de sorte que les extrémités distales et proximales des endoprothèses peuvent se déployer à une vitesse uniforme après la libération du cathéter.

6. Cathéter (20, 60) selon l'une quelconque des revendications précédentes, dans lequel lesdits fils de commande (40, 66) sont construits à partir d'un métal ou d'un polymère souple et élastique comportant une raideur suffisante pour commander la résistance au déploiement des endoprothèses (50, 62) et présentent la structure adaptée à commander le déploiement des endoprothèses, pour maintenir l'espacement de l'endoprothèse et la position en rotation et faciliter le retrait des endoprothèses sans entrave.

7. Cathéter (20, 60) selon l'une quelconque des revendications précédentes dans lequel les fils de commande (40, 66) comprennent des tubes creux souples pouvant être commandés pneumatiquement ou hydrauliquement pour faire varier leur rigidité leur raideur.

8. Cathéter (20, 60) selon l'une quelconque des revendications précédentes comprenant une tige interne (28, 68) à laquelle une pointe avant (36, 70) est fixée à l'extrémité distale du dispositif.
